# EUROPÄISCHE PATENTSCHRIFT

(11) **EP 1 979 033 B1**
(45) Veröffentlichungstag und Bekanntmachung des Hinweises auf die Patenterteilung: **26.10.2011**
(21) Anmeldenummer: 07702629.2
(22) Anmeldetag: 09.01.2007
(51) Int. Cl.: A61M 25/00, A61M 25/06, A61M 39/04, A61M 39/06, A61M 39/24

(54) **KATHETERVORRICHTUNG**
CATHETER DEVICE
SYSTEME DE CATHETER

(30) Priorität: 10.01.2006 DE 102006001255
(43) Veröffentlichungstag der Anmeldung: 15.10.2008
(73) Patentinhaber: B. Braun Melsungen AG, 34212 Melsungen (DE)
(72) Erfinder: FUCHS, Jürgen, 34308 Bad Emstal (DE); NAGEL, Stefan, 16761 Stolpe-Süd (DE)
(74) Vertreter: Klingseisen, Franz
(86) Internationale Anmeldenummer: PCT/EP2007/000120
(87) Internationale Veröffentlichungsnummer: WO 2007/080095

(56) Entgegenhaltungen:
- EP-A- 0 369 314
- EP-A1- 0 268 480
- EP-A1- 1 002 553
- WO-A-00/53254
- WO-A-97/17998
- WO-A-03/002182
- US-A- 4 468 224
- US-A- 5 195 980
- US-A- 5 405 323
- US-A1- 2005 203 543

## Beschreibung

Die Erfindung betrifft eine Kathetervorrichtung und insbesondere einen Arterienkatheter.

Zur invasiven arteriellen Druckmessung ist es bei der so genannten Seldinger-Technik bekannt, zunächst die Arterie mit einer Hohlnadel zu punktieren und dann durch die Hohlnadel einen Führungsdraht einzuführen, worauf nach Herausziehen der Hohlnadel ein Katheter längs des Führungsdrahtes in die Arterie eingeführt wird, an dessen proximalem Ende das Druckmesssystem angeschlossen wird.

Nach dem Herausziehen des Führungsdrahtes aus dem eingeführten Katheter kann Blut aus dem Katheter austreten, bis das Druckmesssystem angeschlossen ist. Wenn der Katheter - meist aus Hygienegründen - gewechselt werden soll, ist das Austreten von größeren Mengen von Blut unvermeidlich. Dabei wird nach Lösen des Druckmesssystems erneut ein Führungsdraht durch den Katheter eingeführt, wobei bereits Blut austritt, worauf nach Herausziehen des Katheters ein neuer Katheter längs des Führungsdrahtes eingeführt und der Führungsdraht aus dem Katheter herausgezogen wird. Bis ein Verschluss oder ein Versorgungsgerät, beispielsweise eine Spritze, an dem proximalen Ende des neu eingeführten Katheters angeschlossen ist, tritt Blut aus.

Eine Kathetervorrichtung der im Oberbegriff des Anspruchs 1 angegebenen Art ist aus US 5 405 323 A bekannt, wobei das Ventil in den Katheteransatz integriert ist. Das Verschlusselement des Ventils ist als Entenschnabelventil mit einem Schlitz ausgebildet, durch den von der proximalen Seite aus ein röhrenförmiger Ansatz eines Ventilöffners hindurchschiebbar ist.

Aus US 5 195 980 ist ein Y-förmiges Anschlusselement mit einem Ventil bekannt, durch das zum Einführen eines Katheters und irgend eines anderen Instruments wie beispielsweise eines Führungsdrahts ein röhrenförmiger Ventilöffner durch ein Entenschnabelventil gedrückt werden kann.

Der Erfindung liegt die Aufgabe zugrunde, ohne großen apparativen Aufwand und ohne umständliche Handhabung an der Kathetervorrichtung einen Blutaustritt zu verhindern, wenn nach Herausziehen des Führungsdrahtes aus dem Katheter ein Versorgungsgerät am Katheter angeschlossen wird.

Diese Aufgabe wird erfindungsgemäß dadurch gelöst, dass das proximale Ende des Kathetersansatzes durch einen biegsamen Schlauch mit einem Ventil verbunden ist, durch dessen Verschlusselement der Führungsdraht von der distalen Seite aus hindurchgeführt werden kann, sodass unter Verhinderung eines Blutaustritts durch das Verschlusselement der Führungsdraht aus der Kathetervorrichtung herausgezogen werden kann.

Vorzugsweise ist das Verschlusselement derart ausgebildet, dass der Führungsdraht auch von der proximalen Seite aus durch das Verschlusselement eingeführt werden kann, sodass in einfacher Weise ein Katheterwechsel vorgenommen werden kann, ohne dass dabei Blut austritt.

Vorteilhafterweise besteht das Ventilverschlusselement aus einer mit einem Schlitz versehenen elastischen Dichtscheibe, die das proximale Ende des Katheters verschließt und durch dessen Schlitz ohne nennenswerten Blutaustritt der Führungsdraht eingeführt und herausgezogen werden kann.

Dadurch, daß das Ventil über einen Verbindungsschlauch mit dem Katheteransatz verbunden ist, kann das Ventil bequem mit der Hand gehalten werden, wenn ein Versorgungsgerät angeschlossen oder ein Führungsdraht eingeführt wird.

Nach einer vorteilhaften Ausgestaltung ist in dem Ventilgehäuse auf der proximalen Seite der Dichtscheibe ein mit einer zentralen Durchtrittsöffnung versehener Ventilkörper in Achsrichtung verschiebbar geführt, der in seiner zurückgeschobenen Stellung mit seiner der Dichtscheibe zugewandten Vorderseite in einem Abstand vor der Dichtscheibe liegt und diese durch Druck auf den geschlitzten Bereich öffnet, wenn ein Druck auf die proximale Seite des Ventilkörpers ausgeübt wird.

Die Erfindung wird beispielsweise anhand der Zeichnung näher erläutert. Es zeigen
- Fig. 1: eine Ansicht eines Arterienkatheters mit Ventil in der Bereitstellung,
- Fig. 2: Ansichten einer Dichtscheibe,
- Fig. 3: eine vergrößerte Darstellung des Ventils in der Schließstellung, und
- Fig. 4: das Ventil mit Anschlussstück in der Offenstellung.

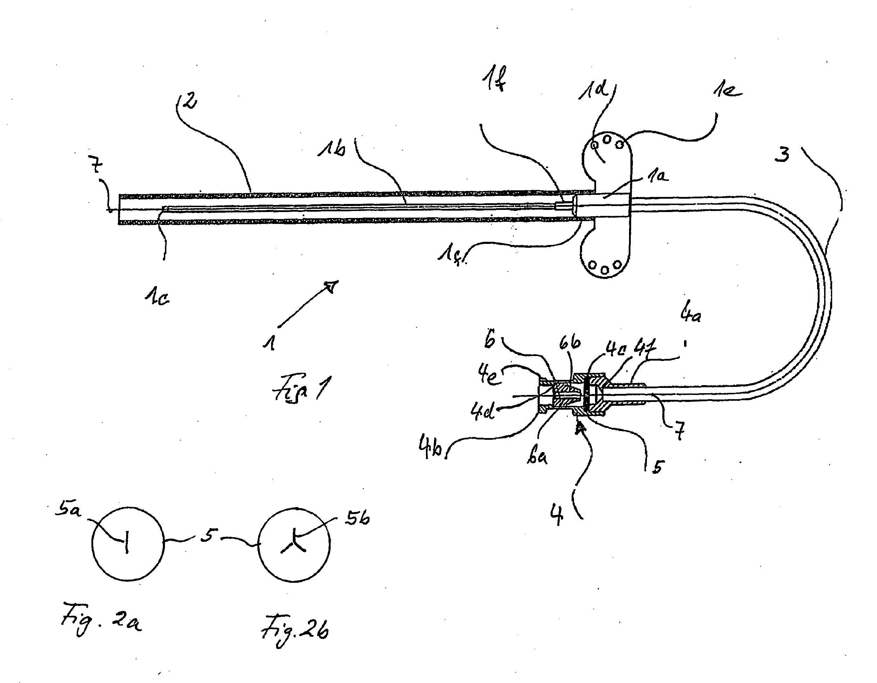

Fig. 1 zeigt einen Arterienkatheter 1 in der Bereitstellung, wobei auf einem Katheteransatz 1a eine Schutzhülse 2 aufgeschoben ist, die einen Kapillarkatheter 1b umgibt, der mit einer sich verjüngenden Spitze 1c versehen ist. Auf diametral gegenüberliegenden Seiten ist der Katheteransatz 1a mit Fixierflügeln 1d versehen, an denen Befestigungslöcher 1e ausgebildet sind. Der distale Abschnitt des Katheteransatzes 1 a bildet einen Schutzhülsensitz 1 g, der in einen verjüngten zylindrischen Abschnitt 1f übergeht, der einen Knickschutz für den Kapillarkatheter 1b bildet.

Am proximalen Ende ist der Katheteransatz 1a mit einem biegsamen Schlauch 3 fest verbunden, an dessen proximalem Ende ein Ventil 4 angebracht ist. Das Gehäuse des Ventils 4 ist bei dem dargestellten Ausführungsbeispiel zweiteilig ausgebildet, wobei ein einen Schlauchansatz bildendes Gehäuseteil 4a, an dem der Schlauch 3 befestigt ist, in ein etwa zylindrisch ausgebildetes, proximales Gehäuseteil 4b derart eingeschraubt oder eingeschweißt ist, dass sich zwischen beiden Gehäuseteilen eine Ringnut 4c ergibt, in der eine Dichtscheibe 5 aus elastischem Material eingesetzt und gehalten ist.

Fig. 2a zeigt in einer Stirnansicht eine Dichtscheibe 5, die in der Mitte mit einem diametral verlaufenden Schlitz 5a versehen ist, der durch Druck in Achsrichtung auf die Dichtscheibe 5 geöffnet werden kann. Fig. 2b zeigt eine andere Ausführungsform mit sternförmig angeordneten Schlitzen 5b, wobei bei dem dargestellten Ausführungsbeispiel drei Schlitze 5b in gleichmäßigen Abständen verteilt ausgebildet sind.

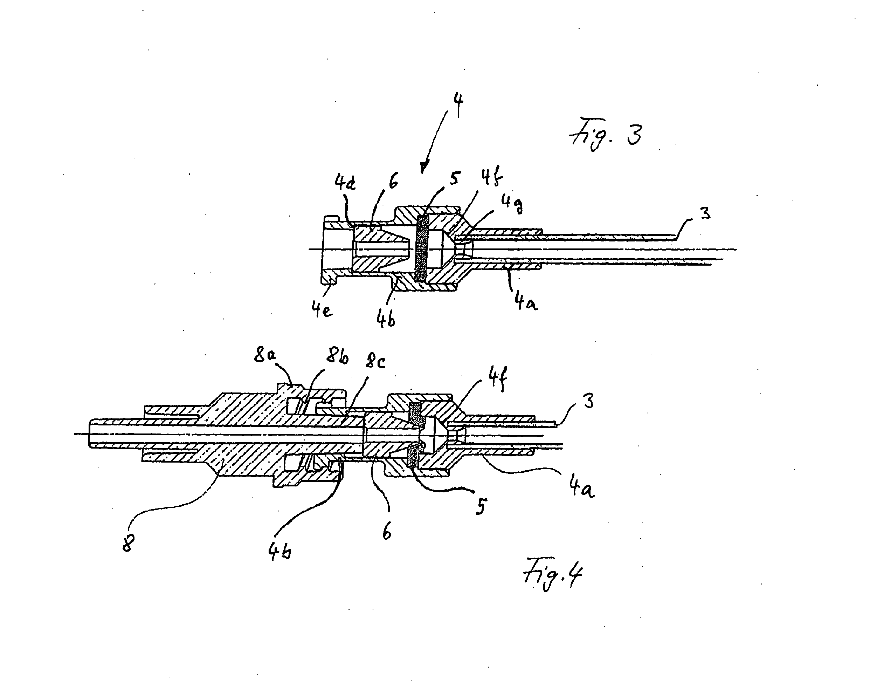

Auf der proximalen Seite der Dichtscheibe 5 ist im Ventilgehäuse bzw. im Gehäuseteil 4b ein im Wesentlichen zylindrischer Ventilkörper 6 in Achsrichtung verschiebbar, der mit einer mittigen Durchtrittsöffnung 6a und auf der distalen Seite mit einem sich kegelstumpfförmig verjüngenden Abschnitt 6b versehen ist. In der in Fig. 1 und 3 dargestellten Stellung liegt der Ventilkörper 6 an einem Absatz 4d des Gehäuseteils 4b an, wobei sein distales, im Durchmesser verjüngtes Ende sich in einem Abstand von der Dichtscheibe 5 befindet. Dieser Ventilkörper 6 dient als Ventilöffner und er kann durch Verschieben in Achsrichtung derart gegen die Dichtscheibe 5 gedrückt werden, dass sich deren Schlitzbereich durch Verformung der Dichtscheibe öffnet und ein Fluid hindurchtreten kann.

Das Gehäuseteil 4b ist auf dem Außenumfang mit einem Gewindeabschnitt 4e versehen, auf den ein Anschlussstück 8 aufgeschraubt werden kann, wie Fig. 4 zeigt, dessen innerhalb einer Muffe 8a mit Innengewinde 8b vorstehender Konus 8c gegen den Ventilkörper 6 drückt und diesen in die Öffnungsstellung der Dichtscheibe 5 verschiebt. Der Konus 8a des Anschlussstücks liegt dabei einerseits dichtend an dem Ventilkörper 6 stirnseitig an und er kann zur weiteren Abdichtung dichtend am Innenumfang des Gehäuseteils 4b anliegen.

Im Gehäuseteil 4a ist in Fig. 1 ein Absatz ausgebildet, an dessen distaler Seite das Ende des Schlauches 3 anliegt, während auf der proximalen Seite ein trichterförmiger Abschnitt 4f ausgebildet ist, durch den der erweiterte Durchmesser im Bereich der Dichtscheibe 5 im Wesentlichen auf den Innendurchmesser des Schlauches 3 verkleinert wird. Der Schlauch 3 kann einen Innendurchmesser in der Größenordnung der Kapillare 1b haben, er ist jedoch dickwandiger ausgeführt, um die Druckmessung nicht durch Verformung der Schlauchwand zu verfälschen.

Bei der in den Fig. 3 und 4 wiedergegebenen Ausführungsform ist im Gehäuseteil 4a anschließend an den trichterförmigen Abschnitt 4f ein sich in den Schlauch 3 hinein erstreckender Abschnitt 4g ausgebildet, der einen Ansatz zur Aufnahme des Schlauchendes bildet und auf dem Innenumfang mit einer abgerundeten Kontur zur Verbesserung der Drahtführung versehen ist.

In der dargestellten Bereitstellung des Katheters 1 ist ein Führungsdraht 7 in den Katheter eingesetzt, der sich auch durch das Ventil 4 und durch die Dichtscheibe 5 erstreckt. Bevor der Katheter eingesetzt wird, wird dieser Führungsdraht 7 aus dem Katheter entfernt, indem der Führungsdraht entweder am proximalen oder am distalen Ende herausgezogen wird, worauf die Schutzhülse 2 abgenommen wird.

Beim Einführen des Katheters in die Arterie eines Patienten wird zunächst durch eine nicht dargestellte Hohlnadel die Arterie punktierte worauf der Führungsdraht 7 durch die Hohlnadel in die Arterie eingerührt wird. Danach wird die Hohlnadel längs des Führungsdrahtes 7 herausgezogen und der Katheter 1 auf den Führungsdraht 7 aufgeschoben, bis die Kapillare 1b in die Arterie eingeführt ist. Hierbei dringt das proximale Ende des Führungsdrahtes 7 durch den Schlitz 5a bzw. durch die Schlitze 5b in der Dichtscheibe 5 und durch den Ventilkörper 6, sodass er am proximalen Ende des Ventil 4 vorsteht, wie dies auch Fig. 1 zeigt. Während des Einführens des Katheters in die Arterie längs des Führungsdrahtes dringt nur wenig Blut in den Katheter, weil das selbsttätig schließende Ventil 4 ein Luftpolster in der Kathetervorrichtung bildet und der Führungsdraht in der Kapillare 1b kaum Blut austreten lässt. Gegen Ende des Einfiihrvorgangs dringt der Führungsdraht 7 durch die Dichtscheibe 5, wobei kein Blut austritt.

Nach dem Einführen der Kapillare 1b in die Arterie wird der Führungsdraht 7 am proximalen Ende aus dem Ventil 4 herausgezogen, worauf ein Druckmesssystem am Gehäuseteil 4b durch Aufschrauben des Anschlussstückes 8 angeschlossen werden kann, durch das der Ventilkörper 6 gegen die Dichtscheibe 5 gedrückt wird, sodass der Durchtritt für die Druckmessung offen ist, wie Fig. 4 zeigt.

In der Zeit zwischen Herausziehen des Führungsdrahtes 7 und Anschließen des Druckmesssystems kann bei der bekannten Kathetervorrichtung ohne Ventil Blut austreten, weil die punktierte Arterie unter Druck steht. Das Druckmesssystem muss deshalb möglichst schnell angeschlossen werden, um den Blutaustritt gering zu halten. Dagegen besteht bei der erfindungsgemäßen Ausgestaltung mit dem selbständig schließenden Ventil keine Gefahr eines Blutaustritts, sodass das Bedienungspersonal in Ruhe das Druckmesssystem oder eine andere Versorgungseinrichtung anschließen kann.

Nach Beendigung der Druckmessung kann das Druckmesssystem vom Gehäuseteil 4b gelöst werden, ohne dass die Gefahr besteht, dass Blut aus dem Ventil 4 austritt, weil die Dichtscheibe 5 selbsttätig den Durchtritt verschließt. Dabei wird der Ventilkörper 6 durch die sich in die Schließstellung verformende elastische Dichtscheibe 5 zurückgeschoben. Der Ventilkörper 6 kann danach an der Dichtscheibe anliegend verbleiben, sodass er nicht in die in Fig. 1 wiedergegebene Stellung zurückgeschoben zu werden braucht.

Arterienkatheter verbleiben häufig längere Zeit im Patienten, wobei aus Hygienegründen zur Vermeidung von Keimbildung der Arterienkatheter ausgewechselt wird. Wenn der Katheter durch einen Neuen ersetzt werden soll, wird zunächst der Führungsdraht 7 durch das Ventil 4 und den Katheter 1 eingeführt, wobei das distale Drahtende die elastische Dichtscheibe 5 nur leicht verformt, wenn sich der Draht 7 durch den Schlitz 5a bzw. 5b der Dichtscheibe 5 schiebt. Auch hierbei liegt das elastische Material der Dichtscheibe 5 an dem Drahtumfang derart an, dass kein Blut austritt.

Nach Einführen des Führungsdrahtes 7 in die in Fig. 1 wiedergegebene Stellung wird der Katheter 1 mit Ventil 4 längs des Führungsdrahtes 7 herausgezogen und ein neuer Katheter 1 mit Ventil 4 auf den Führungsdraht 7 aufgeschoben, wobei das proximale Ende des Führungsdrahtes 7 die Dichtscheibe 5 durchdringt, die wegen des Abstandes des Ventilkörpers 6 von der Dichtscheibe 5 bzw. wegen der Verschiebbarkeit des Ventilkörpers 6 in proximale Richtung etwas verformbar ist. Nach dem Einführen des neuen Katheters 1 kann der Führungsdraht 7 wieder herausgezogen werden, wobei das Ventil automatisch einen Blutaustritt verhindert, worauf nach Bedarf ein Versorgungsgerät oder eine Spritze am Ventilgehäuse angeschlossen werden kann, ohne dass es weiterer Maßnahmen bedürfte, um einen Blutaustritt zu verhindern. Dadurch, dass keinerlei Manipulation an der Verschlussvorrichtung in Form des selbsttätig schließenden Ventils erforderlich ist, ergibt sich eine sichere und einfache Handhabung der Kathetervorrichtung, ohne dass die Gefahr des Austretens von Blut besteht..

Die Verwendung eines biegsamen Schlauches 3 zwischen Katheter 1 und Ventil 4 erleichtert die Handhabung beim Anschließen von Versorgungsgeräten oder bei der Druckmessung.

Der Ventilkörper 6 kann auf der proximalen Seite mit einer sich trichterförmig verjüngenden Durchtrittsöffnung 6a versehen sein, damit das Einführen des Führungsdrahtes 7 von der proximalen Seite aus erleichtert wird. Hierbei dient auch der trichterförmige Abschnitt 4f zum sicheren Einführen des Drahtes 7 in den Schlauch 3.

Die Durchtrittsöffnung 6a des Ventilkörpers 6 mündet im Schlitzbereich der Dichtscheibe 5, sodass der Draht 7 beim Einführen von der proximalen Seite aus selbsttätig in den Schlitzbereich eingerührt wird und der Durchtritt von der Bedienungsperson nicht gesucht werden muss. Nach Durchdringen der Dichtscheibe 5 wird der Draht 7 durch den trichterförmigen Abschnitt 4f zuverlässig in den Schlauch 3 eingeführt.

Die Durchtrittsöffnung an dem trichterförmigen Abschnitt 4f liegt ebenfalls im Schlitzbereich der Dichtscheibe 5, sodass beim Einschieben des Führungsdrahtes 7 von der distalen Seite aus in das Ventil 4 das Drahtende durch die Durchtrittsöffnung des trichterförmigen Abschnitts 4f gezielt auf den Schlitzbereich gelenkt wird, den das Drahtende ohne großen Widerstand durchstoßen kann. Vorzugsweise wird der Abstand zwischen Durchtrittsöffilung des trichterförmigen Abschnitts 4f und der Dichtscheibe 5 wie auch der Abstand zwischen Dichtscheibe 5 und distalem Ende des Ventilkörpers 6 so eng ausgelegt, dass die erforderliche Verformung der Dichtscheibe 5. noch möglich ist, die anschließende Durchtrittsöffnung aber so nahe an der Dichtscheibe 5 liegt, dass die Führung des Drahtes 7 durch den Schlitzbereich der Dichtscheibe 5 begünstigt wird.

Es ist auch möglich, ein anderes Verschlusselement als die beschriebene Dichtscheibe 5 aus elastischem Material vorzusehen, beispielsweise ein federbeaufschlagtes Verschlusselement, das nur von der distalen Seite aus durch das Drahtende soweit geöffnet werden kann, dass beim Einführen des Katheters 1 längs des Führungsdrahtes 7 dessen proximales Ende durch das Ventil 4 nach außen tritt, damit nach der Positionierung des Katheters 1 der Führungsdraht 7 am proximalen Ende herausgezogen werden kann. Bei einer solchen vereinfachten Ausführungsform eines Verschlusselementes wird ein Katheterwechsel nicht begünstigt, bei dem der Führungsdraht 7 auch von der proximalen Seite aus durch das Ventil 4 eingeführt werden muss.

Bei einer vereinfachten Ausführungsform des Ventils 4 kann auch der Ventilkörper 6 entfallen, wenn die Dichtscheibe 5 durch den innenliegenden Konus eines Luer-Anschlusses in die Offenstellung verformbar ist.

## Patentansprüche

1. Kathetervorrichtung, umfassend
eine Katheterkapillare (1b) am distalen Ende eines Katheteransatzes (1a) und
ein selbsttätig schließendes Ventil (4),
**dadurch gekennzeichnet,**
**dass** der Katheteransatz (1a) am proximalen Ende durch einen biegsamen Schlauch (3), dessen Wandstärke dicker ist als die der Katheterkapillare (1b), mit dem Ventil (4) verbunden ist, in dem ein Verschlusselement (5) so ausgebildet ist, dass von der distalen Seite aus ein Führungsdraht (7) durch das Verschlusselement geschoben werden kann.

2. Kathetervorrichtung nach Anspruch 1, wobei das Verschlusselement (5) im Ventil (4) so ausgebildet ist, dass von beiden Seiten ein Führungsdraht (7) hindurchgeschoben werden kann.

3. Kathetervorrichtung nach Anspruch 2, wobei das Verschlusselement als elastische Dichtscheibe (5) mit wenigstens einem mittigen Schlitz (5a) ausgebildet ist.

4. Kathetervorrichtung nach Anspruch 3, wobei die elastische Dichtscheibe (5) in der Mitte sternförmig angeordnete Schlitze (5b) aufweist.

5. Kathetervorrichtung nach Anspruch 3 oder 4, wobei in dem Ventil (4) ein Ventilkörper (6) mit einer Durchtrittsöffnung (6a) in Achsrichtung verschiebbar ist, mittels dem die Dichtscheibe (5) in eine Offenstellung verformbar ist.

6. Kathetervorrichtung nach einem der vorhergehenden Ansprüche, wobei am proximalen Ende des Ventil (4) ein Gewindeabschnitt (4e) für den Anschluss eines Versorgungsgerätes ausgebildet ist.

7. Kathetervorrichtung nach einem der vorhergehenden Ansprüche, wobei das Ventil (4) zweiteilig ausgebildet ist und zwischen den beiden Gehäuseteilen (4a, 4b) die Dichtscheibe (5) gehalten ist.

8. Kathetervorrichtung nach einem der vorhergehenden Ansprüche, wobei im Ventil und/oder am Ventilkörper (6) trichterförmige Abschnitte (4f) zur Erleichterung des Einführens des Führungsdrahtes (7) in den Schlitzbereich der Dichtscheibe (5) ausgebildet sind.

## Claims

1. Catheter device comprising
a catheter capillary (1b) at the distal end of a catheter hub (1a) and
an automatically closing valve (4),
**characterised in that**
the catheter hub (1a) is joined at the proximal end to the valve (4) by means of a flexible tube (3) whose wall thickness is thicker than that of the catheter capillary (1b), and a closing element (5) is embodied in the valve (4) such that a guide wire (7) can be displaced through the closing element from the distal side.

2. Catheter device according to claim 1, wherein the closing element (5) is embodied in the valve (4) such that a guide wire (7) can be displaced therethrough from both sides.

3. Catheter device according to claim 2, wherein the closing element is embodied as a resilient seal disc (5) with at least one middle slot (5a).

4. Catheter device according to claim 3, wherein the resilient seal disc (5) has star-like slots (5b) disposed in the middle.

5. Catheter device according to claim 3 or 4, wherein in the valve (4), a valve body (6) having a throughhole (6a) is displaceable in the axial direction, by means of which the seal disc (5) is deformable into an open position.

6. Catheter device according to one of the preceding claims, wherein at the proximal end of the valve (4) a threaded portion (4e) is formed for connecting a supply apparatus.

7. Catheter device according to one of the preceding claims, wherein the valve (4) is embodied in two parts and the seal disc (5) is held between the two housing parts (4a, 4b).

8. Catheter device according to one of the preceding claims, wherein in the valve and/or at the valve body (6), funnel-shaped sections (4f) are formed for facilitating the insertion of the guide wire (7) into the slot area of the seal disc (5).

## Revendications

1. Dispositif de cathéter, comportant
un capillaire de cathéter (1b) à l'extrémité distale d'un embout de cathéter (1a) et une valve à fermeture autonome (4),
**caractérisé en ce que** l'embout de cathéter (1a) est relié, à l'extrémité proximale, par un tuyau pliable (3), dont l'épaisseur de paroi est supérieure à celle du capillaire de cathéter (1b), à la valve (4), dans laquelle un élément de fermeture (5) est formé de manière à pouvoir faire glisser, à partir du côté distal, au travers de l'élément de fermeture, un fil de guidage (7).

2. Dispositif de cathéter selon la revendication 1, dans lequel l'élément de fermeture (5) dans la valve (4) est formé de manière à pouvoir faire glisser, à partir des deux côtés, un fil de guidage (7).

3. Dispositif de cathéter selon la revendication 2, dans lequel l'élément de fermeture est formé en tant que disque d'étanchéité élastique (5) ayant au moins une fente médiane (5a).

4. Dispositif de cathéter selon la revendication 3, dans lequel le disque d'étanchéité élastique (5) présente au milieu des fentes disposées sous forme d'étoile (5b).

5. Dispositif de cathéter selon la revendication 3 ou 4, dans lequel, dans la valve (4), un corps de valve (6) ayant une ouverture de passage (6a) est coulissant en direction axiale, grâce auquel le disque d'étanchéité (5) peut être déformé dans une position ouverte.

6. Dispositif de cathéter selon l'une quelconque des revendications précédentes, dans lequel, à l'extrémité proximale de la valve (4), une section à filetage (4e) est formée en vue du raccordement à un appareil d'alimentation.

7. Dispositif de cathéter selon l'une quelconque des revendications précédentes, dans lequel la valve (4) est formée de deux pièces et le disque d'étanchéité élastique (5) est maintenu entre les deux parties du boîtier (4a, 4b).

8. Dispositif de cathéter selon l'une quelconque des revendications précédentes, dans lequel, dans la valve et/ou sur le corps de valve (6), au voisinage des fentes du disque d'étanchéité élastique (5), des tronçons en forme d'entonnoir (4f) sont formés en vue de faciliter l'introduction du fil de guidage (7).
